# EUROPEAN PATENT SPECIFICATION

(11) **EP 2 036 575 B1**
(45) Date of publication and mention of the grant of the patent: **18.08.2010**
(21) Application number: 07425563.9
(22) Date of filing: 12.09.2007
(51) Int. Cl.: A61K 47/06, A61K 31/365, A61K 9/48

(54) **Pharmaceutical compositions for oral use for treating patients affected by obesity**
Pharmazeutische Zusammensetzungen zur oralen Verabreichung bei der Behandlung von Adipositas-Patienten
Compositions pharmaceutiques à administration orale pour traiter des patients touchés par l'obésité

(43) Date of publication of application: 18.03.2009
(73) Proprietor: Mader S.r.l., 20123 Milano (IT)
(72) Inventor: Vandoni, Guido, 20151 Milano (IT); Zaniboni, Heny Cassia, 20144 Milano (IT)
(74) Representative: Cinquantini, Bruno

(56) References cited:
- WO-A-01/19340
- WO-A-01/19378

## Description

### State of the art

Orlistat is an active principle belonging to the therapeutic anti-obesity agents group of formula

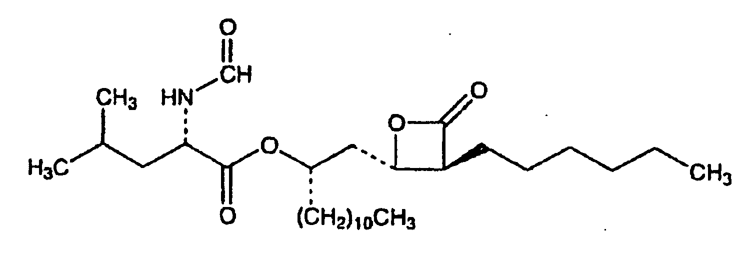

Orlistat is administered in conjunction with low calorie diets for treating patients affected by obesity having a body mass index (BMI) greater than or equal to 30 kg/m², or overweight patients with a BMI ≥ 28 kg/m² with associated risk factors.

Orlistat is a substance which, from the technological viewpoint, has some stability problems; in this respect the active principle Orlistat is low melting (around 44°C) and undergoes rapid hydrolysis in the presence of water or moisture. Stability studies of the raw material, under ICH conditions (25°C, 65% RH) show a 15% degradation of the active principle after 7 days' storage.

Furthermore, degradation is directly proportional to temperature; at a temperature of 40°C and RH of 75% degradation is greater than 40%.

In order to overcome said degradation, formulations in soft capsules have been produced as described in US patent 4,598,089 filed on 18th June 1984 in which the active principle was dissolved in the triglyceride mixture NEOBEE M-5, then distributed into soft gelatin capsules.

Subsequently, in US patent 6,004,996 filed on 6th January 1998, a formulation in granules/pellets was described containing as excipient a stabilizer able to control the degree of humidity and not to degrade the Orlistat. This formulation contains excipients such as: diluents, surfactants and disintegrants.

In US patent 6,358,522 filed on 10th August 1999, formulations in the form of a powder and chewable tablets containing Orlistat combined with a thickening agent and an emulsifier are described.

Capsules comprising Orlistat are furthermore disclosed in WO 01/19340 A and WO 01/19378 A.

### Summary of the invention

The present invention relates to a pharmaceutical formulation based on Orlistat solubilized in mixtures of saturated hydrocarbons obtained from petroleum. These substances due to their lipophilicity do not contain water, the Orlistat being easily solubilized therein. An ionic, non-ionic or amphoteric surfactant is then added to the Orlistat solution obtained.

Surprisingly, the formulation thus attained is found to be stable with an excellent in-vitro dissolution profile.

### Detailed description of the invention

The invention relates to highly stable pharmaceutical preparations for treating obesity which contain Orlistat as the active principle.

The Orlistat is solubilized in mixtures of pharmaceutically acceptable saturated hydrocarbons obtained from petroleum, with an active principle:solvent ratio of between 1:0.5 and 1:10, preferably between 1:1 and 1:2.

Suitable mineral oils are paraffin oil, light paraffin oil, Liquid Paraffin and Paraffin Light Liquid (as described in the European Pharmacopeia). The Paraffin Hard product described in the European Pharmacopeia while having a waxy consistency has also shown an excellent capacity for solubilizing Orlistat, either alone or mixed with other paraffinic oils.

The mineral oil Paraffin Liquid has a relative density of 0.82 - 0.89.

The mineral oil Paraffin Light Liquid has a relative density of 0.810 - 0.875.

The product Paraffin Hard has a melting point of 50 - 61 °C.

The Orlistat solution in saturated hydrocarbon mixtures obtained from petroleum is further supplemented with one or more ionic, non-ionic or amphoteric surfactants in a ratio of active principle:surfactant of between 1:0.001 and 1:1, preferably between 1:0.01 and 1:0.05.

Examples of suitable surfactants are sodium lauryl sulfate, polysorbate 20, 60, 80, alkylamidobetaine and phospholipids, either taken singly or mixed together. Other surfactants useful for implementing the present invention are : Macrogolglycerol ricinoleate, Macrogolglycerol hydroxystearate, Polysorbate 20, Polysorbate 21, Polysorbate 40, Polysorbate 61, Polysorbate 65, Polysorbate 81, Polysorbate 85, lauryl alcohol polyethanoleate, soya, maize or egg lecithins, lecithins purified to the extent of 60, 70, 80, 90 and 95% phosphatidylcholine and 80, 90 and 100% hydrogenated phosphatidylcholine.

The solution of active principle in the paraffinic oil containing the surfactant can be inserted into soft capsules or hard gelatin capsules.

Some purely illustrative examples of the invention are given below with relative solubility and stability data of the prepared products.

### Examples

### Example 1 - Soft capsules

| Active principle: | |
|---|---|
| Orlistat | 120,000 mg |

| Excipients for the fill: | |
|---|---|
| light paraffin oil | 280,140 mg |
| Sodium lauryl sulfate | 2,805 mg |

| Excipients for the shell: | |
|---|---|
| Gelatin | 132,800 mg |
| Glycerol | 77,200 mg |
| Titanium Dioxide | 6,200 mg |
| Capsule characteristics: | 10 oval D |

### Preparation method

### Preparation of the fill

0.350 kg of light paraffin oil were introduced into a stainless steel container and, stirring gently with rotating paddles, 0.0035 kg of sodium lauryl sulfate were added. Stirring was continued until complete dissolution of the surfactant.

The Orlistat (0.15 kg) was added to the solution thus obtained, gently stirring until complete dissolution.

The solution thus obtained was deaerated and maintained under vacuum.

### Preparation of the shell

9.41 kg of purified water was introduced into a container equipped with heated jacket and stirrer, the temperature being controlled at 80°C.

While stirring vigorously the glycerol (6.76 kg) and the gelatin (12.65 kg) were added, to the warmed water until complete dissolution.

The gelatin/glycerol/water solution thus obtained was maintained under vacuum, controlling the temperature at 80°C.

The colouring agent was then added to this solution, prepared by dispersing titanium dioxide (0.59 kg) in glycerol (0.59 kg) with a turboemulsifier.

The titanium dioxide in glycerol dispersion was added to the gelatin/glycerol/water solution to disperse the colouring while maintaining the temperature always at 80°C.

### Encapsulation

By means of an encapsulating machine, the coating mass was placed in the two appropriate hoppers and passed through laminators at 60°C so as to form the coating.

The sheets were moulded through suitable rollers and 403.0 mg of the Orlistat solution were inserted between the two gelatin sheets with a suitable dosing needle.

### Drying

The thus prepared capsules were placed in rotating drying tubes and, after predrying, were positioned on suitable racks in temperature controlled cupboards at 21-22°C and 20% RH for 1-2 days.

### Solubility tests

The dissolution profile was characterized in-vitro in accordance with the following methodology:
- Method: USP
- Apparatus: Paddle II
- Medium: phosphate buffer pH 6.9
- Volume: 900 ml
- Rotation speed: 100 r.p.m
- Temperature: 37°C

### Solubility results (%)

| Sample | After 10' | After 20' | After 30' | After 45' |
|---|---|---|---|---|
| No. 1 | 12.7 | 55.6 | 75.9 | 85.8 |
| No. 2 | 15.2 | 50.7 | 79.9 | 88.7 |
| No. 3 | 11.0 | 60.4 | 80.1 | 86.7 |
| No. 4 | 16.4 | 58.8 | 77.9 | 86.4 |
| No. 5 | 14.4 | 61.1 | 76.1 | 89.3 |
| No. 6 | 14.9 | 60.2 | 80.7 | 95.1 |

The Orlistat-based preparations in soft capsules were subjected to ICH stability studies at conditions of 25°C 60% RH, 30°C 70% RH and 40°C 75% RH and demonstrated excellent active principle stability.

The active principle titres were determined by analysis with HPLC Diode Array MD1510, using 70:30 methanol:water as the mobile phase, with Lichrosphere RP8 column 125x4mm, 5mcm (Merck), at a wavelength of 210 nm.

### Stability results:

| | | | | | | |
|---|---|---|---|---|---|---|
| isothermal 25°C 60% RH | T zero | 1 month | 3 months | 6 months | 9 months | 12 months |
| orlistat HPLC titre | 100.00% | 100.01 % | 100.00% | 100.20% | 100.01 % | 100.2% |
| isothermal 30°C 70% RH | T zero | 1 month | 3 months | 6 months | 9 months | 12 months |
| orlistat HPLC titre | 100.00% | 100.01% | 100.00% | 100.20% | 100.01% | 100.2% |
| isothermal 40°C 75% RH | T zero | 1 month | 3 months | 6 months | | |
| orlistat HPLC titre | 100.00% | 99.90% | 99.80% | 99.90% | | |

### Example 2 - Soft capsules

| Active principle: | |
|---|---|
| Orlistat | 120,000 mg |

| Excipients for the fill: | |
|---|---|
| paraffin oil | 263,000 mg |
| Tween 80 | 20,000 mg |

| Excipients for the capsule: | |
|---|---|
| Gelatin | 132,800 mg |
| Glycerol | 77,200 mg |
| Iron oxide | 6,200 mg |
| Capsule characteristics: | 10 oval D |

### Preparation method

### Preparation of the fill

The paraffin oil (0.263 kg) was weighed and inserted into a stainless steel container and, stirring gently with rotating paddles, Tween 80 (0.020 kg) was added and dissolved. Stirring was continued until complete dissolution of the surfactant.

The Orlistat (0.12 kg) was added to the solution thus obtained and dissolved while gently stirring.

The solution thus obtained was deaerated and maintained under vacuum.

### Preparation of the capsule

9.41 kg of purified water were introduced into a container equipped with heated jacket and stirrer, the temperature being controlled at 80°C.

While stirring vigorously the glycerol (6.76 kg) then the gelatin (12.65 kg) were added to the thus heated water and dissolved.

The gelatin/glycerol/water solution thus obtained was maintained under vacuum and temperature controlled at 80°C.

The colouring agent, prepared by dispersing the iron oxide (0.59 kg) in glycerol (0.59 kg) with a turboemulsifier, was added to this solution.

The iron oxide in glycerol dispersion was added to the gelatin/glycerol/water solution to disperse the colouring while maintaining the temperature at 80°C.

### Encapsulation

By means of an encapsulating machine, the coating mass was placed in the two appropriate hoppers and passed through laminators at 60°C so as to form the coating.

The sheets were passed through suitable rollers to allow moulding and 403.0 mg of the Orlistat solution were inserted between the two gelatin sheets with a suitable dosing needle.

### Drying

The already prepared capsules were placed in rotating drying tubes and, after predrying, were placed onto suitable racks in temperature controlled cupboards at 21-22°C and 20% RH for 1-2 days.

### Example 3 - Hard gelatin capsules

| Active principle: | |
|---|---|
| Orlistat | 120,000 mg |

| Excipients: | |
|---|---|
| Paraffin Liquid | 100,000 mg |
| Paraffin Hard | 60,000 mg |
| Phosphatidylcholine (80) | 20,000 mg |
| Capsule: size 00 white opaque, average weight 118 mg ± 7.0 mg. | |

### Preparation method

### Preparation

The Paraffin Liquid (10.000 kg) and Paraffin Hard (6.000 kg) were weighed, placed in a stainless steel container and melted at a temperature of 70°C, stirring gently with rotating paddles; the phosphatidylcholine (2.000 kg) was then added and melted. Stirring was maintained until this latter dissolved completely, controlling the temperature at 30°C.

The Orlistat (12.00 kg) was added to the solution thus obtained and dissolved while gently stirring.

### Encapsulation

Using a hard capsule filling machine equipped to dispense liquids, the liquid was dispensed into hard gelatin capsules, size 00, to a weight of 300 mg/cps of fill.

## Claims

1. Pharmaceutical compositions for oral use for treating patients affected by obesity, **characterized by** comprising, as the active principle, Orlistat solubilized in mixtures of pharmaceutically acceptable saturated hydrocarbons obtained from petroleum, and one or more surfactants.

2. Compositions as claimed in claim 1 wherein the saturated hydrocarbon mixture is paraffin oil or light paraffin oil.

3. Compositions as claimed in claim 1 wherein the saturated hydrocarbon mixture is the product Paraffin Light Liquid as identified in the European Pharmacopeia (relative density from 0.810 to 0.875).

4. Compositions as claimed in claim 1 wherein the saturated hydrocarbon mixture is the product Paraffin Liquid as identified in the European Pharmacopeia (relative density from 0.82 to 0.89).

5. Compositions as claimed in claim 1 wherein the saturated hydrocarbon mixture is the product Paraffin Hard as identified in the European Pharmacopeia (melting point from 50 to 61 °C).

6. Compositions as claimed in claim 1 wherein the saturated hydrocarbon mixture is a varying proportion mixture of 2 or 3 of the products chosen from the group consisting of paraffin oil, light paraffin oil, Paraffin Light Liquid, Paraffin Liquid and Paraffin Hard in all possible quantitative combinations.

7. Compositions as claimed in claim 1 wherein the surfactants can be ionic, nonionic or amphoteric, as a single surfactant or in a mixture.

8. Compositions as claimed in claim 7 wherein the surfactant is sodium lauryl sulfate alone or mixed with other surfactants.

9. Compositions as claimed in claim 7 wherein the surfactant is chosen from the polysorbate 20, 60, 80 group and mixtures thereof.

10. Compositions as claimed in claim 7 wherein the surfactant is chosen from the alkylamidobetaines group and mixtures thereof.

11. Compositions as claimed in claim 7 wherein the surfactant is chosen from the phospholipids group and mixtures thereof.

12. Compositions as claimed in claim 7 wherein the surfactant, or the surfactant mixture, is chosen from the group consisting of Macrogolglycerol ricinoleate, Macrogolglycerol hydroxystearate, Polysorbate 20, Polysorbate 21, Polysorbate 40, Polysorbate 61, Polysorbate 65, Polysorbate 81, Polysorbate 85, lauryl alcohol polyethanoleate, soya, maize or egg lecithins, lecithins purified to the extent of 60, 70, 80, 90 and 95% phosphatidylcholine and 80, 90 and 100% hydrogenated phosphatidylcholine.

13. Pharmaceutical compositions as claimed in claim 1 enclosed in soft capsules.

14. Pharmaceutical compositions as claimed in claim 1 enclosed in hard gelatin capsules.

15. Compositions as claimed in claim 1 wherein the ratio of Orlistat to the saturated hydrocarbon mixture which acts as solvent is between 1:0.5 and 1:10.

16. Compositions as claimed in claim 14 wherein the ratio of Orlistat to the saturated hydrocarbon mixture which acts as solvent is between 1:1 and 1:2.

17. Compositions as claimed in claim 1 wherein the ratio of active principle solution:surfactant is between 1 :0.001 and 1:1.

18. Compositions as claimed in claim 16 wherein the ratio of active principle solution:surfactant is between 1:0.01 and 1:0.05.

## Patentansprüche

1. Pharmazeutische Zusammensetzungen zur oralen Verwendung zum Behandeln von Patienten, welche unter Fettsucht leiden, **dadurch gekennzeichnet, dass** diese als den aktiven Bestandteil Orlistat enthalten, welches in Mischungen von pharmazeutisch akzeptablen gesättigten, aus Erdöl erhaltenen Kohlenwasserstoffen sowie einem oder mehreren Tensiden solubilisiert ist.

2. Zusammensetzungen nach Anspruch 1, wobei die gesättigte Kohlenwasserstoffmischung Paraffinöl oder leichtes Paraffinöl ist.

3. Zusammensetzungen nach Anspruch 1, wobei die gesättigte Kohlenwasserstoffmischung das Produkt dünnflüssiges Paraffin (Paraffin Light Liquid), wie in der Europäischen Pharmacopoeia (relative Dichte zwischen 0,810 und 0,875) identifiziert, ist.

4. Zusammensetzungen nach Anspruch 1, wobei die gesättigte Kohlenwasserstoffmischung das Produkt flüssiges Paraffin (Paraffin Liquid), wie in der Europäischen Pharmacopoeia (relative Dichte von 0,82 bis 0,89) identifiziert, ist.

5. Zusammensetzungen nach Anspruch 1, wobei die gesättigte Kohlenwasserstoffmischung das Produkt Hartparaffin (Paraffin hard), wie in der Europäischen Pharmacopoeia (Schmelzpunkt zwischen 50 und 61°C) identifiziert, ist.

6. Zusammensetzungen nach Anspruch 1, wobei die gesättigte Kohlenwasserstoffmischung eine Mischung mit variierendem Anteil von 2 oder 3 der Produkte ausgewählt aus der Gruppe bestehend aus Paraffinöl, leichtem Paraffinöl, dünnflüssigem Paraffin, flüssigem Paraffin und Hartparaffin in allen möglichen quantitativen Kombinationen ist.

7. Zusammensetzungen nach Anspruch 1, wobei die Tenside, als ein einzelnes Tensid oder in einer Mischung, ionisch, nicht ionisch oder amphoter sein können.

8. Zusammensetzungen nach Anspruch 7, wobei das Tensid Natriumlaurylsulfat alleine oder vermischt mit anderen Tensiden ist.

9. Zusammensetzungen nach Anspruch 7, wobei das Tensid aus der Polysorbat 20-, 60-, 80-Gruppe und Mischungen hiervon ausgewählt ist.

10. Zusammensetzungen nach Anspruch 7, wobei das Tensid aus der Alkylamidobetaingruppe und Mischungen hiervon ausgewählt ist.

11. Zusammensetzungen nach Anspruch 7, wobei das Tensid aus der Phospholipidgruppe und Mischungen hiervon ausgewählt ist.

12. Zusammensetzungen nach Anspruch 7, wobei das Tensid oder die Tensidmischung aus der Gruppe ausgewählt ist, welche aus Macrogolglycerolricinoleat, Macrogolglycerolhydroxystearat, Polysorbat 20, Polysorbat 21, Polysorbat 40, Polysorbat 61, Polysorbat 65, Polysorbat 81, Polysorbat 85, Laurylalkoholpolyethanoleat, Soja-, Mais- oder Ei-Lecithinen, Lecithinen gereinigt bis zu einem Ausmaß von 60, 70, 80, 90 und 95 % Phosphatidylcholin und 80, 90 und 100 % hydriertem Phosphatidylcholin besteht.

13. Pharmazeutische Zusammensetzungen nach Anspruch 1, welche in weichen Kapseln eingeschlossen sind.

14. Pharmazeutische Zusammensetzungen nach Anspruch 1, welche in harten Gelatinekapseln eingeschlossen sind.

15. Zusammensetzungen nach Anspruch 1, wobei das Verhältnis von Orlistat zu der gesättigten Kohlenwasserstoffmischung, welche als Lösungsmittel agiert, zwischen 1:0,5 und 1:10 beträgt.

16. Zusammensetzungen nach Anspruch 14, wobei das Verhältnis von Orlistat zu der gesättigten Kohlenwasserstoffmischung, welche als Lösungsmittel agiert, zwischen 1:1 1 und 1:2 beträgt.

17. Zusammensetzungen nach Anspruch 1, wobei das Verhältnis von aktiver Bestandteil-Lösung : Tensid zwischen 1:0,001 und 1:1 beträgt.

18. Zusammensetzungen nach Anspruch 16, wobei das Verhältnis von aktiven Bestandteil-Lösung : Tensid zwischen 1:0,01 und 1:0,05 beträgt.

## Revendications

1. Compositions pharmaceutiques destinées à une utilisation orale pour le traitement de patients affectés par l'obésité, **caractérisées en ce qu'**elles comprennent, en tant que principe actif, de l'orlistat solubilisé dans des mélanges d'hydrocarbures saturés pharmaceutiquement acceptables dérivés de pétrole, et un ou plusieurs surfactants.

2. Compositions selon la revendication 1, dans lesquelles le mélange d'hydrocarbures saturés est de l'huile de paraffine ou de l'huile de paraffine légère.

3. Compositions selon la revendication 1, dans lesquelles le mélange d'hydrocarbures saturés est le produit Paraffin Light Liquid tel qu'identifié dans la Pharmacopée européenne (densité relative comprise entre 0,810 et 0,875).

4. Compositions selon la revendication 1, dans lesquelles le mélange d'hydrocarbures saturés est le produit Paraffin Liquid tel qu'identifié dans la Pharmacopée européenne (densité relative comprise entre 0,82 et 0,89).

5. Compositions selon la revendication 1, dans lesquelles le mélange d'hydrocarbures saturés est le produit Paraffin Hard tel qu'identifié dans la Pharmacopée européenne (point de fusion compris entre 50 et 61°C).

6. Compositions selon la revendication 1, dans lesquelles le mélange d'hydrocarbures saturés est un mélange à proportions variables de 2 ou 3 des produits choisis dans le groupe constitué par l'huile de paraffine, l'huile de paraffine légère, Paraffin Light Liquid, Paraffin Liquid et Paraffin Hard dans toutes les combinaisons quantitatives possibles.

7. Compositions selon la revendication 1, dans lesquelles le surfactant peut être ionique, non ionique ou amphotère, sous la forme d'un surfactant seul ou dans un mélange.

8. Compositions selon la revendication 7, dans lesquelles le surfactant est le sodium lauryl sulfate seul ou mélangé à d'autres surfactants.

9. Compositions selon la revendication 7, dans lesquelles le surfactant est choisi dans le groupe constitué par les polysorbates 20, 60, 80 et les mélanges de ceux-ci.

10. Compositions selon la revendication 7, dans lesquelles le surfactant est choisi dans le groupe constitué par les alkylamidobétaïnes et les mélanges de celles-ci.

11. Compositions selon la revendication 7, dans lesquelles le surfactant est choisi dans le groupe constitué par les phospholipides et les mélanges de ceux-ci.

12. Compositions selon la revendication 7, dans lesquelles le surfactant, ou le mélange de surfactants, est choisi dans le groupe constitué par le ricinoléate de macrogolglycérol, l'hydroxystéarate de macrogolglycérol, le Polysorbate 20, le Polysorbate 21, le Polysorbate 40, le Polysorbate 61, le Polysorbate 65, le Polysorbate 81, le Polysorbate 85, le polyéthanoléate d'alcool laurylique, les lécithines de soja, de maïs ou d'oeuf, les lécithines purifiées à hauteur de 60, 70, 80, 90 et 95 % de phosphatidylcholine et 80, 90 et 100 % de phosphatidylcholine hydrogénée.

13. Compositions pharmaceutiques selon la revendication 1, dans des gélules molles.

14. Compositions pharmaceutiques selon la revendication 1, dans des gélules en gélatine dure.

15. Compositions selon la revendication 1, dans lesquelles le rapport d'orlistat sur le mélange d'hydrocarbures saturés qui agit en tant que solvant est compris entre 1/0,5 et 1/10.

16. Compositions selon la revendication 14, dans lesquelles le rapport d'orlistat sur le mélange d'hydrocarbures saturés qui agit en tant que solvant est compris entre 1/1 et 1/2.

17. Compositions selon la revendication 1, dans lesquelles le rapport de la solution de principe actif/surfactant est compris entre 1/0,001 et 1/1.

18. Compositions selon la revendication 16, dans lesquelles le rapport de la solution de principe actif/surfactant est compris entre 1/0,01 et 1/0,05.
